# EUROPEAN PATENT APPLICATION

(11) **EP 2 561 802 A2**
(43) Date of publication of application: **27.02.2013**
(21) Application number: 12180438.9
(22) Date of filing: 14.08.2012
(51) Int. Cl.: A61B 5/00, G08B 21/02, A61G 7/018, G06Q 50/24, G06F 19/00

(54) **Occupant support suite and method for responding to an acoustic signature of a stand alone device**

(30) Priority: 22.08.2011 US 201113214467
(71) Applicant: Hill-Rom Services, Inc., Batesville, IN 47006-9167 (US)
(72) Inventor: Penninger, Jason A, Indianapolis, IN 46220 (US); Hornbach, David W, Brookville, IN 47012 (US); O'Keefe, Christopher R, Batesville, IN 47006 (US); Receveur, Timothy Joseph, Guilford, IN 47022 (US)
(74) Representative: Findlay, Alice Rosemary

(57) **Abstract**

An occupant support suite comprises a bed **(20)** an acoustic sensor **(110, 110A)** associated with the bed for receiving an acoustic signature **(160)** corresponding to a stand alone device **(150)** and a processor **(112, 112A)** for comparing the signature to a library **(116)** of reference signatures, and, if the comparison is satisfactory, issuing a notification **(208)** to the bed, to an off-bed destination **(210)** or to both.

## Description

The subject matter described herein relates to occupant supports, such as hospital beds, and to a method of responding to an acoustic signature, such as an alarm or alert, issued by a stand alone device.

Hospital beds and similar occupant or patient supports are often used in conjunction with items of medical equipment that are not components of the bed itself. Examples if these devices include occupant condition monitoring devices such as heart monitors, respiration monitors, pulse oximeters, and thermometers, as well as therapeutic devices such as respirators and sequential compression devices (SCD's) that apply periodically varying compression to an occupant's lower extremities to prevent deep vein thrombosis.

The occupant condition monitoring devices are designed to issue an audible alarm if the parameter detected by the device is "out of bounds", for example if the occupant's heart rate exceeds an upper limit or if the oxygen saturation of the occupant's blood falls below a lower limit. The condition monitoring and therapeutic devices may also be designed to issue an audible alarm in the event of certain equipment malfunctions, for example if a respirator hose becomes disconnected or a heart monitor becomes disconnected from an electrical outlet. In addition a therapeutic device may be designed to issue an audible alert, which is less intense form of an alarm, when the device completes a commanded therapy session. For example an SCD device may be user programmable to operate for a prescribed period of time or a prescribed number of compression cycles after which it issues an alert to advise the attending caregiver staff that the therapy has been completed. The device may cease operation automatically upon completion of the prescribed therapy or could be designed to continue operating until a caregiver intervenes.

The audible alarm emitted by the monitoring or therapeutic device could be continuous or could be interrupted by periods of silence, similar to Morse code. Either way the alarm has a distinctive acoustic output related to its amplitude and frequency content and/or due to the sound/silence pattern of an interrupted signal. The device in question may offer the caregiver a selection of different alarm acoustic outputs so that he or she can distinguish among alarms issued by different types of equipment (e.g. a respirator as opposed to a heart monitor) or distinguish among different alarms issued by the same device (e.g. between an elevated heart rate and a depressed heart rate) or distinguish among different pieces of substantially identical equipment (e.g. between two different SCD pumps each serving a different occupant of a different bed in the same room). The devices may also offer the caregiver a selection of alarm volumes either as an intrinsic element of the selected alarm acoustic output or as an independently specifiable attribute of the selected alarm.

One shortcoming of the device alarms arises from the fact that the devices are not connected to a facility communication network such as a nurse call system. A member of the caregiver staff is unlikely to be in the immediate vicinity of the equipment and the affected patient at the moment the equipment alarm sounds. Instead the members of the staff are more likely to be occupied with other patients at other locations or on duty at a remote nurse's station. As a result the members of the caregiver staff may not be within earshot of the equipment issuing the alarm.
Moreover, because the equipment is not a component of the bed, the alarm cannot cause the bed to undergo any change of state or configuration that might be beneficial for taking whatever action might be necessary to respond to the alarm. Instead, there will be an additional lapse of time after the caregiver arrives at bedside while the caregiver assesses the nature of the alarm and, if appropriate, adjusts the bed to a more desirable configuration.

A method for responding to an acoustic output issued by a stand alone device and caused by a defined condition comprises receiving an acoustic signature corresponding to the acoustic output of the stand-alone device and causing the occupant support to undergo a change of state from an initial state causally unrelated to the defined condition to a changed state.

A method for responding to an acoustic output issued by a stand alone device used in conjunction with an occupant support comprises receiving an acoustic signature corresponding to the acoustic output of the stand-alone device, determining if the received acoustic signature compares satisfactorily to a reference signature and, if the acoustic signature compares satisfactorily to a reference signature, issuing a notification to the occupant support, to an off-support destination or to both.

An occupant support suite comprises a bed, an acoustic sensor associated with the bed for receiving an acoustic signature emitted by a stand alone device, and a processor for a) comparing the received signature to a library of one or more reference signatures, and b) in the event the received signature compares satisfactorily to one of the reference signatures, issuing a notification to the bed, or to an off-bed destination, or to both.

The invention will now be further described by way of example with reference to the accompanying drawings, in which:
FIG. **1** is a schematic, side elevation view of an occupant support suite comprising an occupant support or bed, an acoustic sensor and a processor.
FIG. **2** is a schematic end elevation view taken in the direction **2--2** of FIG. **1****.**
FIG. **3** is a block diagram depicting certain steps in a method of responding to an acoustic output of a stand alone device.
FIG. **4** is a schematic diagram showing certain elements of the processor of FIG. 1**.**

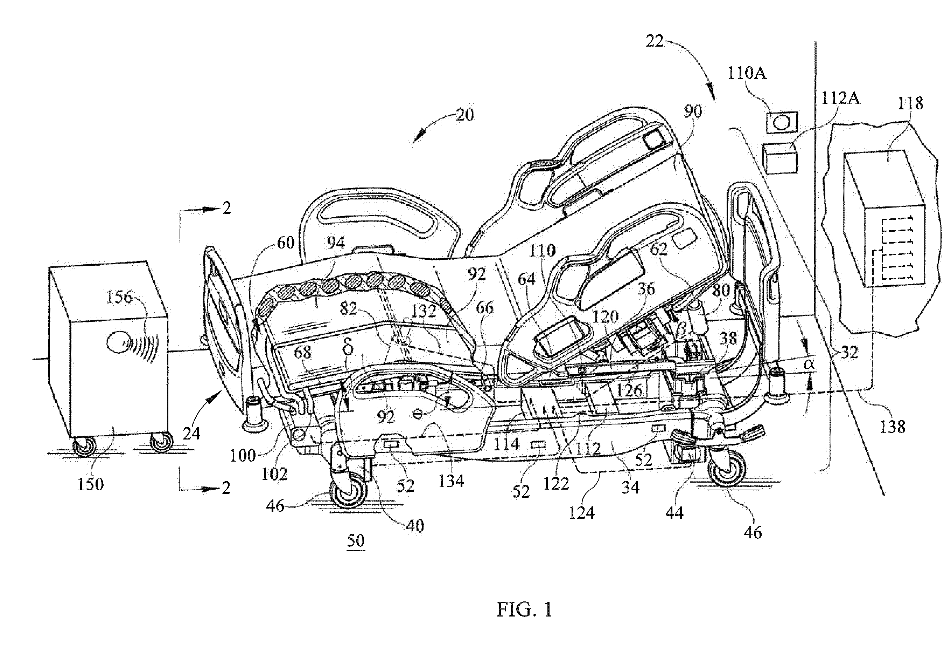

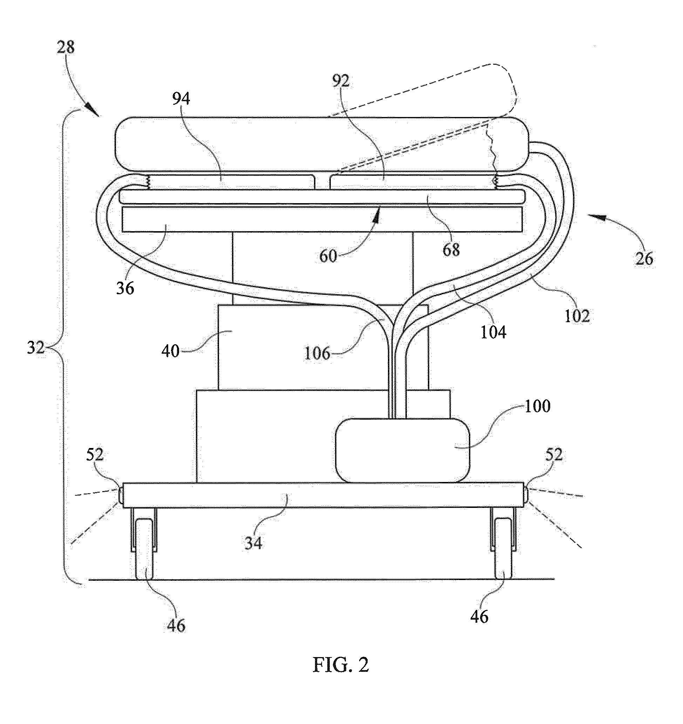

Referring to FIGS. **1** and **2** an occupant support in the form of a hospital bed **20** extends longitudinally from a head end **22** to a foot end **24** and laterally from a left side **26** to a right side **28.** The bed includes a framework **32** comprising a base frame **34** and an elevatable frame **36.** A lift system, represented in part by head end and foot end canister lifts **38, 40,** renders the elevatable frame height adjustable relative to the base frame and makes the base frame adjustable to a head down (Trendelenberg) inclination or a foot down (reverse Trendelenberg) inclination as indicated by inclination angle **α.** Each canister lift includes an actuator such as electric motor **44** and associated mechanical elements connected between the elevatable frame and the base frame for operating the elevatable frame, e.g. for raising and lowering the elevatable frame relative to the base frame. Casters **46** extend from the base frame to floor **50**. A set of lights **52** is provided to illuminate the floor in the vicinity of the bed.

Framework **32** also includes a segmented deck **60** comprised of a head or upper body deck segment **62**, a seat deck segment **64,** a thigh deck segment **66** and a calf deck segment **68** all supported on the elevatable frame. Linear actuators **80, 82** connect the elevatable frame and selected deck segments for operating the segments, specifically for adjusting the angular orientations **β, θ,** and **δ** of the upper body, thigh and calf segments.

Bed **20** also includes a mattress such as air mattress **90,** a pair of turn bladders **92, 94,** and a pump **100** which serves as an actuator for operating the mattress and turn bladders. Hose **102** connects pump **100** to mattress **90** so that the mattress can be inflated and pressurized by air provided by the pump. The mattress can be inflated to a pressure suitable for sustained support of an occupant (the sustained use pressure) or can be inflated to a higher prescribed pressure which provides a stiffer support useful for cardiopulmonary resuscitation. The pump can also deliver a sequence of pressure pulses to mattress **20** to provide percussion and vibration (P&V) therapy to a bed occupant. Hoses **104, 106** connect pump **100** to the turn bladders so that the bladders can be selectively inflated and pressurized. **FIGS. 1-2** employ solid lines to show the bladders deflated. FIG. **2** also employs broken lines to show the left bladder, which is in the form of a bellows, inflated as it would be to assist a caregiver's efforts to turn a bed occupant to his or her right. The turn bladders can also be inflated and deflated, out of phase with each other, to gently and continually rock the occupant laterally back and forth, an operation known as continuous lateral rotation therapy (CLRT). A caregiver can use a control panel, not illustrated, to enter instructions for CLRT to occur for a prescribed interval of time, for a prescribed number of cycles, or until some other form of intervention is invoked to command cessation of the therapy.

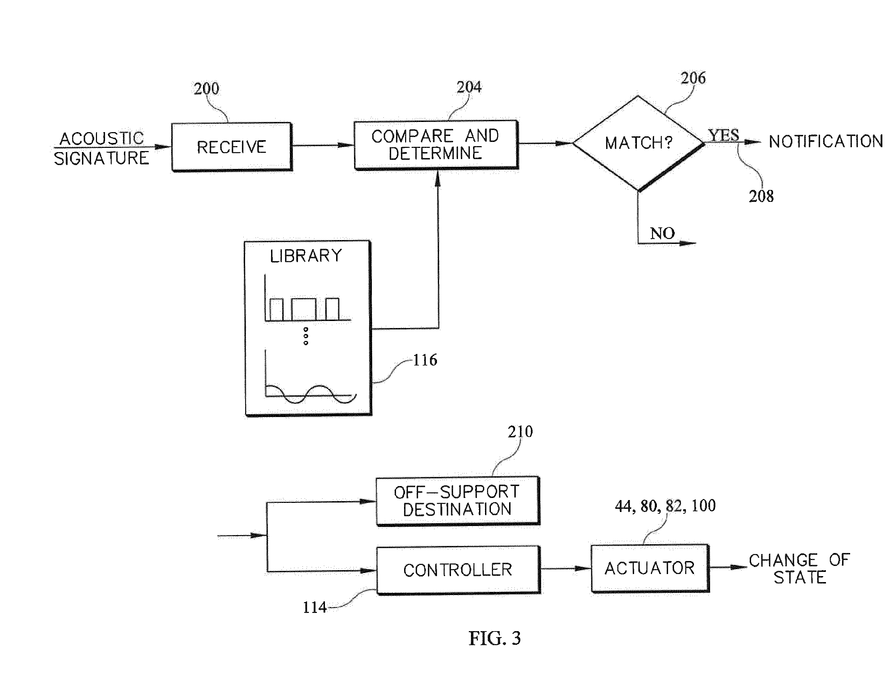

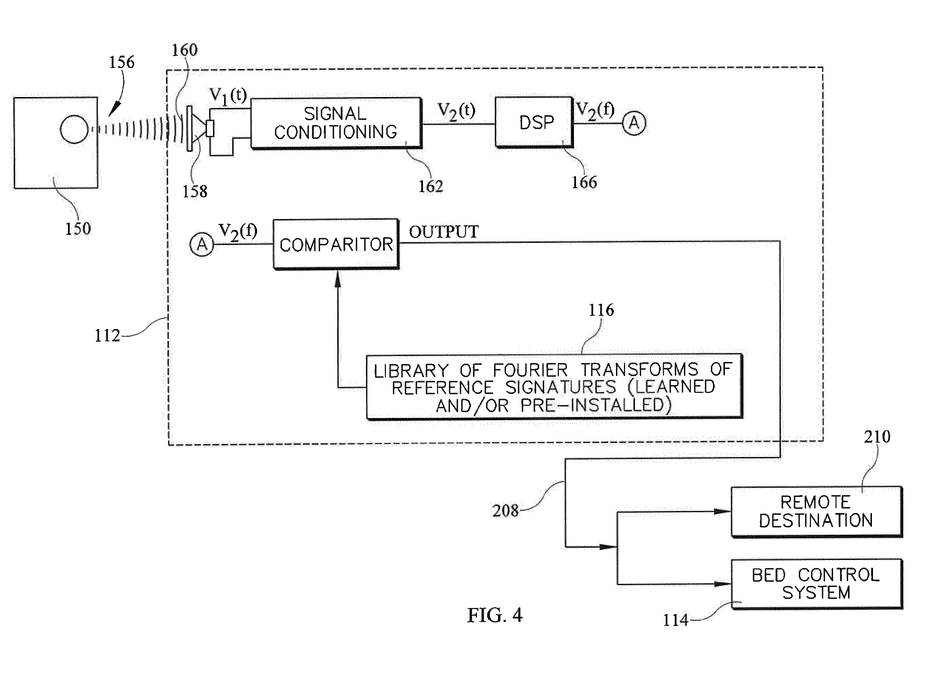

The bed also includes an acoustic sensor **110,** a processor **112,** and a bed controller or control circuitry **114.** The processor includes a library **116** (FIGS. **3-4**) of one or more reference acoustic signatures or equivalent representations of the acoustic signatures, such as their Fourier transforms. The processor is operable in a learning mode in which a user can load reference acoustic signatures into the library. The processor is also operable in an armed or operational mode in which it compares an acoustic signature of interest to the members of the library to determine if the signature of interest matches one of the library members.

Acoustic sensor **110** and processor **112** are associated with the bed by virtue of being mounted thereon. Processor **112** is in communication with acoustic sensor **110** and with controller **114** by way of communication paths **120, 122.** The controller also communicates with, among other things, actuator motor **44,** linear actuators **80, 82,** pump **100,** and a facility communication network **118,** such as a hospital nurse call system, by way of communication paths **124, 126, 132, 134, 138.** The illustration suggests wired communication paths however other communication techniques such as optical and non-optical wireless techniques are also applicable.

FIG. **1** shows the acoustic sensor and processor mounted on the bed and suggests a physical coupling to each other by way of communication pathway **120.** An alternative arrangement is an occupant support suite comprising bed **20,** acoustic sensor **110A** at an off-bed location, such as affixed to the wall of the room and/or a processor **112A** also at an off-bed location. That is, the acoustic sensor and processor need not be components of the bed and need not rely on a physical communication pathway in order to be associated with the bed.

FIG. **1** also shows a stand alone device **150** used in conjunction with bed **20.** The device is referred to as stand-alone because it is not a component of the bed, is not connected to the bed by electrical wires, and is intended to be moved from place to place, independently of the bed, as required to satisfy the needs of patients throughout the hospital. A device qualifies as a stand-alone device even though it may be capable of being temporarily placed on or mounted on the bed, for example, by hooks or easily installable and removable connectors. The device is acoustically connected to the bed. When the acoustic sensor is mounted on the bed (e.g. sensor **110**) the connection is a direct acoustic connection. When the acoustic sensor is mounted elsewhere, such as on the wall (e.g. sensor **110A**) the connection is an indirect acoustic connection.

Examples of stand alone devices include heart monitors, respiration monitors, oximeters, and body temperature monitors, all of which monitor physiological parameters of a bed occupant. Other examples include equipment respirators and SCD pumps, both of which apply therapy to a bed occupant. The devices are configured to emit an audible acoustic output **156** in response to a defined condition. For example if the device is a heart monitor it may be designed to emit an audible alarm output if the monitor perceives that the heart rate of the patient being monitored is not within defined upper and lower limits or if the patient's heartbeat is consistent with an abnormality such as ventricular tachycardia. A second example is a respirator designed to emit an alarm if an air tube extending from the respirator to the patient becomes disconnected. As already noted the audible output could be continuous or could be interrupted by periods of silence, similar to Morse code. Either way the emitted acoustic signal has a distinctive acoustic output related to its amplitude and frequency content and/or due to the sound/silence pattern of an interrupted signal. The device in question may offer the caregiver a selection of acoustically different alarms so that he or she can distinguish among alarms issued by different types of devices (e.g. a respirator as opposed to a heart monitor) or distinguish among different alarms issued by the same device (e.g. between an elevated heart rate and a depressed heart rate) or distinguish among two or more substantially identical devices (e.g. two different SCD pumps each serving a different occupant of a different bed in the same room). The devices may also offer the caregiver a selection of volumes either as an intrinsic element of the selected alarm or as an independently specifiable attribute of the selected alarm.

In practice a user engages the processor's learning mode and exposes the processor to a one or more alarm signatures corresponding to the alarm outputs of the stand alone devices expected to be used in conjunction with the bed. By doing so the user "teaches" the processor a family of signatures associated with the alarms. The teaching process enables the processor to associate each signature to which it is exposed with, for example, a particular type of device, different types of alarms generated by a particular device, or different alarms generated by two or more examples of a particular device depending on the degree of specificity the user introduces into the teaching process. Alternatively or additionally the processor may include a pre-installed library of predefined alarm acoustic signatures to which it is expected to be exposed.

Referring additionally to FIGS. **3-4****,** in operation, certain defined conditions will result in the stand alone device emitting an alarm, i.e. an acoustic output **156.** The defined conditions include physiological conditions of the occupant, for example an abnormal heartbeat, and conditions of the stand alone device such as a disconnected respirator hose or an interruption of electrical power. At block **200** microphone **158** or other acoustic sensor **110** or **110A** associated with the bed receives a corresponding acoustic signature **160** which may differ from acoustic output 156 due to, for example, attenuation of the signal amplitude. The microphone converts the signature to an output voltage V₁(t). As seen in FIG. **3** the processor operates on the received signature, e.g. on voltage V₁(t), by passing it to a signal conditioner **162** such as an amplifier or filter and passing the resulting conditioned voltage signal V₂(t) to a digital signal processor **166** whose output V₂(F) is the Fourier transform of V₂(t). Because the processor converts acoustic signature **158** to the Fourier transform of a voltage, the reference signatures in library **116** are also Fourier transforms of the alarm signatures expected to be encountered.

At blocks **204** and **206** comparitor **168** compares the received acoustic signature to each member of library **116** of acoustic signatures and determines whether or not the received, conditioned signal compares satisfactorily to a member of the library. In particular the processor tests whether or not V₂(F) compares satisfactorily to one of the reference signatures (i.e. whether or not it matches one of the reference signals within some tolerance). As used herein, comparing an acoustic signature to a reference acoustic signature is the same as comparing equivalent representations of the acoustic signature and the reference signature, such as the Fourier transforms of a voltage as in the above example.

If the processor determines that the test signature (e.g. V₂(F) compares satisfactorily to one of the library signatures, the processor issues a notification **208** to an off-bed destination **210,** to the bed (specifically to controller **114**) or to both.

An example off-bed destination **210** for notification **208** is a facility communication network **118** such as a nurse call system or a pager to notify one or more caregivers who are not within earshot of the device alarm that the device has issued an alarm. The content of the message conveyed through the communication system can be as specific as the specificity used during the teaching process or the specificity of any predefined reference signatures installed in the library.

Certain abnormalities related to patient condition, including the patient's physiological condition may indicate that the state of the bed should be changed from its existing physical or operational state to a physical or operational state more compatible with the well-being of a patient occupying the bed or to a physical or operational state more compatible with attending to the condition that caused the alarm. For example, if the notification were issued in response to an acoustic signature that compared satisfactorily to the heartbeat signature of a patient suffering ventricular tachycardia, the notification would provoke the controller to command the appropriate bed actuators (e.g. **44, 80, 82, 100**) to place the bed in its CPR (cardiopulmonary resuscitation) configuration. The CPR configuration is one in which all the deck segments are at a substantially horizontal orientation, the deck is at working height satisfactory for CPR and, if the bed is equipped with an air mattress, the mattress is inflated or pressurized to a prescribed pressure which exceeds a sustained use pressure so that the mattress stiffness exceeds its sustained use stiffness. In another example if the notification were issued in response to an acoustic signature that compared satisfactorily to the signature of a patient suffering low oxygen saturation, the notification would provoke the controller to command the appropriate bed actuators to rotate upper body deck segment **62** to a substantially zero degree orientation angle **β** relative to the elevatable frame. Another example change of state is illuminating lights **52.**

Certain abnormalities related to a condition of the stand alone device may also indicate that the state of the bed should be changed from its existing or initial physical or operational state, which is not or is not known to be causally related to the defined condition that triggered the alarm, to a physical or operational state more compatible with the well-being of a patient occupying the bed or to a physical or operational state more compatible with attending to the condition that caused the alarm. For example, if the notification were issued in response to an acoustic signature that compared favorably to the signature corresponding to a respirator hose having become disconnected, the notification would cause the controller to command the appropriate bed actuators (e.g. pump **100**) to change the state of the bed by discontinuing any ongoing operation, for example CLRT or P&V therapy, that might have been underway at the time notification **208** was issued. In the case of a cyclic operation such as CLRT the discontinuance could be delayed until completion of the current cycle or until the bed attained a state compatible with the well-being of a patient occupying the bed or with facilitating caregiver intervention. Another example change of state is illuminating lights **52.**

The following table provides a matrix of stand alone devices, abnormal conditions that might be associated with each device, and possible responses thought to be compatible with patient well-being and/or with facilitating caregiver intervention. The entries in each cell of the matrix are nonexhaustive.

| Device | Abnormal Condition | Response |
|---|---|---|
| Heart Monitor | Rapid or slow pulse rate; ventricular tachycardia | Place bed in CPR configuration |
| | | Summon Nurse |
| Respiration Monitor | Rapid or slow respiration; sleep apnea. | Rotate bed upper body section to 0 degrees orientation |
| | | Summon Nurse |
| Respirator | Supply tube disconnected, electrical power interrupted, abnormally high or low pressure. | Discontinue CLRT and P&V therapy |
| | | Summon nurse |
| SCD Pump | Hose disconnected, abnormally low pressure | Summon Nurse |
| Pulse Oximeter | Low oxygen saturation | Rotate bed upper body section to 0 degrees orientation |
| | | Summon nurse |
| Thermometer | abnormally high or low body temperature | Summon nurse |

In some circumstances the step of determining if the received acoustic signature compares satisfactorily to a reference signature may be unnecessary. One possible example is if it were desired to accommodate only a single acoustic signature. In that case the step of causing the occupant support to undergo a change of state could be carried out as a result of having detected an acoustic signal without first having to compare the signal to a reference signal and determine if the signals match each other within some tolerance.

Although this disclosure refers to specific embodiments, it will be understood by those skilled in the art that various changes in form and detail may be made.

## Claims

1. A method for responding to an acoustic output issued by a stand alone device, the device being used in conjunction with an occupant support, the method comprising:
receiving an acoustic signature corresponding to the acoustic output of the stand-alone device, the output having been generated in response to a defined condition; and
causing the occupant support to undergo a change of state from an existing state causally unrelated to the defined condition to a changed state.

2. The method of claim 1 wherein the defined condition is selected from the group consisting of a physiological condition of the occupant and a condition of the device.

3. The method of each of claim 1 or claim 2 wherein the change of state comprises discontinuing an operation of the occupant support.

4. The method of any preceding claim wherein the causing step is preceded by a step of determining if the received acoustic signature compares satisfactorily to a reference signature belonging to a set of one or more reference signatures.

5. The method of claim 4 wherein the determining step includes comparing Fourier transforms of the received acoustic signature and the reference signature to each other.

6. The method of claims 4 or 5, the method further comprising issuing a notification to one or both of the occupant support and an off-support destination if the acoustic signature compares satisfactorily to one of the reference signatures.

7. The method of claim 6 wherein the off-support destination comprises a communication network.

8. An occupant support suite comprising:
a bed;
an acoustic sensor for receiving an acoustic signature emitted by a stand alone device; and
a processor for
a) comparing the received signature to a library of reference signatures, and
b) in the event the received signature compares satisfactorily to one of the reference signatures, issuing a notification to the bed or to an off-support destination or both.

9. The occupant support suite of claim 8 wherein the bed includes a framework having at least one operable component, an actuator connected to the operable component and operable to operate the component, and a controller coupled to the actuator to command operation of the actuator in response to the notification.

10. The occupant support suite of claim 9 wherein the received signature corresponds to a perceived physiological abnormality of an occupant of the bed and wherein the controller commands the actuator to place the bed in a state compatible with attending to a patient exhibiting the physiological abnormality.

11. The occupant support suite of claim 10 wherein the framework includes a frame and a segmented deck comprised of deck segments supported on the frame, and the compatible state is one in which the deck segments are at a substantially horizontal orientation.

12. The occupant support of claim 10 wherein the bed includes an air mattress and the compatible state is one in which the mattress is inflated to a prescribed pressure which exceeds a sustained use pressure.

13. The occupant support suite of claim **10** wherein the framework includes an orientation adjustable upper body deck segment supported on a frame member and wherein the compatible state is one in which the upper body deck segment is at a substantially zero degree orientation relative to the frame member.

14. The occupant support suite of claim **9** wherein the received signature corresponds to a perceived equipment abnormality and wherein the controller commands the actuator to discontinue an ongoing operation.

15. The occupant support suite of any one of claims 8 to 14 wherein the off support destination comprises a communication network.
